# EUROPEAN PATENT SPECIFICATION

(11) **EP 3 969 113 B1**
(45) Date of publication and mention of the grant of the patent: **17.09.2025**
(21) Application number: 20726038.1
(22) Date of filing: 12.05.2020
(51) Int. Cl.: A61K 8/34, A61K 8/60, A61K 31/7004, A61K 31/702, A61K 31/7016, A61K 9/00, A61K 9/06, A61K 9/08, A61K 9/12, A61K 31/047, A61K 47/10, A61Q 17/00, A61P 17/00, A61P 31/04

(54) **USE OF A SUGAR OR SUGAR ALCOHOL**
VERWENDUNG VON ZUCKER ODER ZUCKERALKOHOL
UTILISATION D'UN SUCRE OU D'UN ALCOOL DE SUCRE

(30) Priority: 16.05.2019 EP 19174808
(43) Date of publication of application: 23.03.2022
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: APPAVOO, Shanthi, Whitefield, Bangalore 560 066 (IN); DASGUPTA, Anindya, Whitefield, Bangalore 560 066 (IN); MAJUMDAR, Amitabha, Whitefield, Bangalore 560 066 (IN); MALLEMALA, Prathyusha, Whitefield, Bangalore 560 066 (IN); SALGAONKAR, Neha, Whitefield, Bangalore 560 066 (IN)
(74) Representative: van den Brom, Coenraad Richard
(86) International application number: PCT/EP2020/063131
(87) International publication number: WO 2020/229446

(56) References cited:
- WO-A1-2017/173244
- WO-A1-2017/201296
- WO-A1-2020/052916
- WO-A2-2013/122931
- CA-A1- 2 881 267
- GB-A- 2 498 543
- US-A1- 2013 115 610
- WANG YANHAN ET AL: "Staphylococcus epidermidisin the human skin microbiome mediates fermentation to inhibit the growth ofPropionibacterium acnes: implications of probiotics in acne vulgaris", APPLIED MICROBIOLOGY AND BIOTECHNOLOGY, SPRINGER BERLIN HEIDELBERG, BERLIN/HEIDELBERG, vol. 98, no. 1, 22 November 2013 (2013-11-22), pages 411 - 424, XP035329006, ISSN: 0175-7598, [retrieved on 20131122], DOI: 10.1007/S00253-013-5394-8

## Description

### Field of the invention

The present invention relates to use of a sugar or a sugar alcohol for protection of skin against harmful bacteria. The present invention is especially useful in formulating compositions comprising sugar alcohols like glycerol which act as prebiotics by skin commensal bacteria like *S*. *epidermidis* to produce metabolites like lactic acid which by way of the present invention has been shown to inhibit growth of harmful bacteria like E. *Coli, S. Aureus* among others. The present invention is especially useful in that there is no need to use conventional antimicrobial actives which may destroy the microbiome of the skin especially in those of babies.

### Background of the invention

Skin in mammals are considered as one of the largest organs in the body, especially one which has the largest surface area. Skin forms the first line of defence against microorganisms which may invade the body though the air, water, food or material that come in contact with the body. When the body is infected on the skin or systemically, traditional approach to such hygiene problems has been to treat the skin/ body with antimicrobial actives that reduce or kill the germs. Recent research indicates that a lot of the bacteria that permanently reside on the skin (called skin commensal bacteria) do not actually cause infections, rather they are beneficial bacteria that protect the skin against disease causing pathogens. Several mechanisms have been proposed to explain the protection and some of the popular ones are: physically occupying space on skin to prevent colonization of pathogens; producing metabolites that ward of harmful possibly pathogenic organisms; generating metabolites that strengthen the innate defence mechanisms to prevent infection by harmful pathogens; and providing other benefits such as maintaining skin pH, barrier function etc. Thus, of late, there is a trend in moving away from the approach of treating the skin with broad spectrum antimicrobial actives to kill all microoganisms present on skin (or any other part of the body) as a means of treating infections. Rather, the approach is more towards targeted or selective inhibition/ killing of the desired microorganism to the exclusion of the skin commensal organism. This ensures that the skin microbiome is maintained in a healthy balanced state for long term hygiene and health.

The present inventors, in looking to solve the problem of selective kill of harmful bacteria on skin like *E.coli* and S. *aureus* directed their research to boosting the number and functionality of skin commensal bacteria through use of prebiotics or Selective Fermentation Inducers (SFI)

They found, during the course of the research, that S. *epidermidis* present on skin can ferment glycerol and other sugars and sugar alcohols and produce metabolites which inhibit the growth of such pathogens. To the knowledge of the present inventors the use of glycerol and similar prebiotics is not known for inhibiting such type of bacteria.

It is known that sugars and sugar alcohols act as prebotics for skin commensal bacteria e.g. WO2017/201296 discloses that a prebiotic compound e.g glycerol, sucrose, maltose, lactose, and carbohydrates and other components support fermentation and growth of *S*. *Epidermidis.* The present inventors believe that it is not known that such metabolites of *S*. *Epidermidis* which feed on prebiotics like glycerol are produced in sufficient quantities on skin to kill or inhibit harmful bacteria like *E*. *Coli, S. Aureus, P Aeruginosa, or P acnes.* They further believe that it is an added advantage that the metabolites thus produced while killing the harmful bacteria do not affect the commensal bacterial in any negative way.

Another prior art document (Wang et al., 2013) discloses that Staphylococcus epidermidis in the human skin microbiome mediates fermentation to inhibit the growth of Propionibacterium acnes. It is thus an object of the present invention to provide for a composition for therapeutic use in inhibiting growth of harmful microorganisms on the skin.

### Summary of the invention

The first aspect of the present invention relates to sugar or sugar alcohol, wherein the sugar is selected from monosaccharides glucose, fructose, galactose, or disaccharides sucrose, lactose; or sugar alcohols glycerol, lactitol, for use in therapeutically inhibiting growth of harmful bacteria while nourishing and growing the skin commensal bacteria, wherein the harmful bacteria are selected from *E. coli, S*. *aureus* or mixtures thereof, wherein the sugar or sugar alcohol is a prebiotic for the skin commensal bacterium to produce lactic acid, wherein the skin commensal bacterium is S. *epidermidis.*

It is particularly preferred that the sugar or sugar alcohol is glycerol.

A preferred aspect of the present invention relates to the sugar or sugar alcohol for therapeutic use in a composition suitable for application to the skin.

The present invention also relates to the sugar or sugar alcohol for therapeutic use in a skin care composition as a promoter of antipathogenic metabolite secretion by skin commensal bacteria.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The present invention relates to a sugar or sugar alcohol for therapeutic use in protecting skin against harmful bacteria. Further, the present invention relates to the sugar or sugar alcohol for therapeutic use in a skin care composition as a skin protecting agent. Further, it relates to the sugar or sugar alcohol for therapeutic use in a skin care composition as commensal bacteria nourishing agent. Alternatively, the present invention also relates to the sugar or sugar alcohol for therapeutic use in a skin care composition as prebiotic for commensal bacterium.

Any preference described hereinbelow with regard to one aspect of the invention (e.g. the composition or the sugar or sugar alcohol or the therapeutic use according to the invention) is also preferred for use in any one of the other aspects of the invention.

The present invention relates to the sugar or sugar alcohol for therapeutic use in protecting skin from harmful bacteria via nourishing the skin commensal bacteria wherein the sugar is selected from monosaccharides glucose, fructose, galactose; or disaccharides sucrose, lactose; or sugar alcohols glycerol, lactitol. Preferred sugars are selected from glucose, fructose, galactose, sucrose or lactose, more preferred sugars are selected from fructose or sucrose.

For instance, it is particularly preferred that the sugar alcohol is glycerol.

It is also preferred that the sugar or sugar alcohol is for therapeutic use in a composition also comprising a surfactant, an emollient, or humectant or mixtures thereof.

The invention thus also provides the sugar or sugar alcohol (preferably glycerol) for the therapeutic use in a skin care or skin cleansing composition as a skin microbiome balancing agent.

"Skin" as used herein is meant to include skin on any part of the body (e.g. face, neck, chest, back, arms, underarms, hands, legs, buttocks and scalp). It is especially useful for protecting the skin of babies. By babies is meant a child whose age is less than five years preferably less than three years more preferably less than a year. It is to be understood that the benefits disclosed and claimed by the sugar or sugar alcohol in the present invention could also be achieved by a composition comprising the sugar or sugar alcohol of the present invention. Such a composition could be in the form of a leave-on composition. Alternatively and equally preferably it could be delivered through a wash-off format for delivering selective protective benefit to topical areas e.g. skin and/or hair of mammals, especially humans. Such a composition includes any product applied to a human body for also improving appearance, cleansing, or general aesthetics. The composition of the present invention may be delivered with a topically acceptable carrier which could be an anhydrous base, liquid, lotion, cream, foam, scrub, gel, or emulsion.

The sugar or sugar alcohol as per this invention is a prebiotic for a skin commensal bacterium. The skin commensal bacteria is S. *epidermidis.* The skin is protected as per this invention from harmful bacteria, being *E. coli, S. aureus,* or mixtures thereof.

By a sugar is meant a compound which may be a monosaccharide, a disaccharide, an oligosaccharide or a complex carbohydrate. Monosaccharides which may be included in the composition are glucose, fructose, galactose, or combinations thereof. Disaccharides which may be included are sucrose, lactose, or combinations thereof. Sugar alcohols include polyhydric alcohols i.e. compounds which are sugar based alcohols having multiple hydroxyl groups. The sugar alcohols for use in obtaining the benefit of the present invention are glycerol, lactitol or combinations thereof. Most preferred sugar alcohol for use in the present invention is glycerol which is also known as glycerine.

The invention, as per a preferred aspect provides for the sugar or sugar alcohol for therapeutic use in a composition suitable for application to the skin.

The sugar or sugar alcohol for use in the present invention is preferably included in such a composition at a level of from 0.1 to 80%, preferably 0.5 to 50, further more preferably 0.5 to 20% by weight of the composition. The composition may be in the form of a leave on composition or a wash off composition.

The composition of the invention is preferably a wash-off composition and this is enabled by including 1 to 80% by weight of a surfactant. In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used but preferred surfactant is of the anionic or non-ionic type.

The pH of a wash off composition as per the present invention is in the range of 5 to 11, preferably in the range of 5.5 to 10.

The surfactant may be a soap. Soap is a suitable surfactant for personal washing applications of composition of the invention. The soap is preferably C8-C24 soap, more preferably C10-C20 soap and most preferably C12-C16 soap. The soap may or may not have one or more carbon-carbon double bond or triple bond. The cation of the soap may be alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc.

A typical fatty acid blend consisted of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions. The most preferred soap is a laurate soap. The soap, when present in solid forms of the present invention is present in an amount of 30 to 90%, preferably from 50 to 85%, more preferably 55 to 75% by weight of the composition. The soap, when present in liquid forms of the composition is present in 0.5 to 20%, preferably from 1 to 10% by weight of the composition.

Alternatively the surfactants are non-ionic surfactants, such as C8-C22, preferably C8-C16 fatty alcohol ethoxylates, comprising between 1 and 8 ethylene oxide the surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16. Suitable surfactant concentrations in liquid forms of cleaning application are generally more than 0.5 but less than 10%, preferably from 1 to 5 % by weight of the composition. In solid compositions, the surfactant is preferably present in 5 to 40%, preferably from 10 to 30% by weight of the composition.

Water may preferably be present in 10 to 90% by weight of the composition depending on the format of the composition. In solid composition water may be present in 10-30%, while in liquid or semi-solid composition, water may be present in 40 to 90%.

When the composition in accordance with the invention is a leave on composition, it preferably comprises one or more surfactant, emollient, humectant, pigment and preservative.

The pH of a leave on composition as per the present invention is in the range of 5 to 9, preferably in the range of 5.5 to 8.

The carrier acts as diluent or dispersant for the ingredients of the compositions. The carrier may be aqueous-based, anhydrous or an emulsion, whereby a water-in-oil or oil-in-water emulsion is generally preferred. If the use of water is desired, water typically makes up the balance of the composition, which most preferably is from 40 to 80 % by weight of the composition.

In addition to water, organic solvents may optionally be included as carrier to assist any other carrier in the compositions of the present invention. Examples include alkanols like ethyl and isopropyl alcohol.

Other suitable organic solvents include ester oils like isopropyl myristate, cetyl myristate, 2-octyldodecyl myristate, avocado oil, almond oil, olive oil and neopentylglycol dicaprate. Typically, such ester oils assist in emulsifying the compositions, and an effective amount is often used to yield a stable, and most preferably, water-in-oil emulsion.

Emollients may also be used, if desired, as a carrier. Alcohols like 1 -hexadecanol (i.e. cetyl alcohol) are preferred. Other emollients include silicone oils and synthetic esters. Silicone oils suitable for use include cyclic or linear polydimethylsiloxanes containing from 3 to 9, preferably from 4 to 5 silicon atoms. Non-volatile silicone oils useful as emollients include polyalkyl siloxanes, polyalkylaryl siloxanes and polyether siloxane copolymers. The non-volatile polyalkyl siloxanes useful polydimethylsiloxanes. Silicone elastomers may also be used. The ester emollients that may optionally be used are:
(i) alkenyl or alkyl esters of fatty acids having 10 to 20 carbon atoms. Examples thereof include isoarachidyl neopentanoate, isononyl isonanonoate, oleyl myristate, oleyl stearate, and oleyl oleate;
(ii) ether-esters such as fatty acid esters of ethoxylated fatty alcohols;
(iii) polyhydric alcohol esters. Ethylene glycol mono and di-fatty acid esters, diethylene glycol mono- and di-fatty acid esters, polyethylene glycol (200- 6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters;
(iv) wax esters such as beeswax, spermaceti, stearyl stearate and arachidyl behenate; and,
(v) sterols esters, of which cholesterol fatty acid esters are examples.

Emollients, when present, typically make up from 0.1 to 50 % by weight of the composition, including all ranges subsumed therein.

Fatty acids having from 10 to 30 carbon atoms may also be included as carriers. Examples of such fatty acids include pelargonic, lauric, myristic, palmitic, stearic, isostearic, oleic, linoleic, arachidic, behenic or erucic acid and mixtures thereof.

Moisturisation may be improved through use of petrolatum or paraffin. Thickeners may also be utilized as a portion of the carrier in the compositions. Typical thickeners include cross-linked acrylates (e.g. Carbopol^{®} 982), hydrophobically-modified acrylates (e.g. Carbopol^{®}1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.001 to 5, optimally from 0.01 to 0.5 % by weight of the composition.

Surfactants may also be present. When present, the total amount of surfactants is 2 to 40 % by weight, and preferably from 4 to 20 % by weight, optimally from 5 to 12 % by weight of the composition. The surfactant is selected from the group consisting of anionic, nonionic, cationic and amphoteric actives. Particularly preferred nonionic surfactants are those with a C₁₀₋₂₀ fatty alcohol or acid hydrophobe condensed with from 2 to 100 moles of ethylene oxide or propylene oxide per mole of hydrophobe; mono- and di- fatty acid esters of ethylene glycol; fatty acid monoglyceride; sorbitan, mono- and di-C8-C20 fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C_{8 to 20} acyl isethionates, acyl glutamates, C_{8 to 20} alkyl ether phosphates and combinations thereof.

Various other ingredients may also be used in compositions. Actives are defined as skin benefit agents other than emollients and other than ingredients that merely improve the physical characteristics of the composition. Although not limited to this category, general examples include extender pigments such as talcs and silicas, as well as alpha- hydroxy acids, beta-hydroxy acids and zinc salts.

Beta-hydroxy acids include salicylic acid. Zinc oxide and zinc pyrithione are examples of useful zinc salts.

Suitable preservatives include alkyl esters of p-hydroxybenzoic acid, hydantoin derivatives, propionate salts, and a variety of quaternary ammonium compounds. Particularly preferred preservatives are methyl paraben, propyl paraben, phenoxyethanol and benzyl alcohol. Preservatives are from 0.1 to 2 % by weight of the composition.

The packaging could be a patch, bottle, tube, roll-ball applicator, propellant driven aerosol device, squeeze container or lidded jar.

Without wishing to be bound by theory the present inventors believe that the sugar or sugar alcohol used in the present invention is a prebiotic for the skin commensal bacteria *S. epidermidis.*

The present invention is therefore intended to increase, promote, improve, maintain or sustain skin health and skin resiliency through improving the barrier health or NMF. This is attained through ensuring a healthy skin ecosystem through balanced microbiome health. By balanced microbiome health is meant that the ratio of commensal bacteria to harmful bacteria is maintained in a desired range for the skin health to be maintained. This invention is especially useful for use on skin of babies where the use of broad spectrum anti bacterial agents are considered too harsh as it tends to interfere with the growth and maintenance of a healthy skin microbiome for long term health of the babies as they grow into adults. It is well accepted that baby skin barrier is more vulnerable than adult skin. Baby skin barrier is more delicate and water loss from the surface is faster than in adult skin. Similarly, the microbiome of baby skin is seen to be different from that of adult skin. Soon after birth, the baby's skin as well as skin microbiome continues to change for up to about three to five years. Together with the barrier function, the microbiome plays a critical role in skin's natural defence. It is thus imperative that the skin microbiome of babies and infants should be nourished to allow for its natural growth and maturation thereby maintaining optimum skin health.

Thus, whether used on skin of babies or others, the composition for use in the present invention is substantially free of a conventional antimicrobial compound. By substantially free is meant that conventional antimicrobial actives are present in less than 0.1wt%, more preferably less than 0.05 wt%, further more preferably less than 0.01wt%, even more further preferably less than 0.001% by weight of the composition. By conventional antimicrobial actives is meant an antimicrobial active which kills or inhibits bacteria which attack skin like E. *coli,S. aureus, P aeruginosa, or P acnes* among others. Preservatives which are included in compositions for their microbial stability are excluded from the definitions of antimicrobial actives mentioned above. Preservatives are included to ensure that the compositions are stable with respect to microorganisms which may grow and degrade the compositions. On the other hand, antibacterial compounds are included in compositions to hinder the growth of microorganisms which are present on the substrate (e.g skin) on which the compositions are applied.

Conventional antimicrobial compounds are generally those from the class of biguanides, bisphenols, halophenols, oligodynamic metal compounds like those of silver or zinc, cationic antimicrobial compounds or essential oil actives. Biguanide has a general base structure which may be further derivatised e.g. chlorhexidine or polyhexamethylene biguanide (PHMB). Bisphenols include triclosan or hexachlorophene. Halophenol include chloroxylenol (PCMX). Cationic compounds are another class of antimicrobial actives e.g. benzalkonium chloride, cetyl pyridinium chloride or cetyl trimethyl ammonium bromide.

The class of low boiling alcohols (which are fast acting antimicrobials) are especially not included in any substantial amount in the composition of the invention. Ideally they are absent from such compositions. By low boiling alcohols are meant monohydric alcohols with 2 to 5 carbon atoms.

When the composition of the invention is formulated for use on skin of babies, it is preferred that it is substantially free of a fragrance component. By a fragrance component is meant a molecule or a group of molecules that are compounded to form a perfume. The characteristic of such compounds is that they volatile (to varying degrees) and offer a pleasant odour. In the context of perfumes or fragrance component, "by substantially free" is meant that that they are present in less than 0.05wt%, more preferably less than 0.01 wt%, further more preferably less than 0.005wt%, even more further preferably less than 0.001% by weight of the composition.

With a goal of developing a range of products suitable for all types of skin especially for nourishing baby skin, this invention took upon an objective to examine various prebiotics for skin commensal bacterial and arrived at sugars and sugar alcohols as claimed (e.g. glycerol) to attain the same.

Glycerol is a common ingredient found in a wide variety of skin cleansing and skin care products. The main function of glycerol is to act as a humectant, thereby keeping skin hydrated and healthy through improved barrier function. Using both in vitro and ex vivo methods, it has been found by way of the present invention that glycerol and other sugars or sugar alcohols can be utilized by skin commensal bacteria as a food source, making glycerol a "skin prebiotic" ingredient. By utilizing glycerol and similar compounds, skin commensal bacteria can produce various fatty acids which are seen to be beneficial for skin. This is an additional benefit beyond the barrier benefits provided by glycerol.

Through extensive experiments, the present inventors observed robust increase in various metabolites like fatty acids, organic acids, and diols on application of the sugars and sugar alcohols on normal skin. Organic acids predominant of which was lactic acid was observed as the model metabolite. Lactic acid is seen to have multiple skin benefits such as hydration, resilience and barrier function. A product which is rich in glycerol therefore would help nourish the skin's especially baby skin's, microbiome as well as improve barrier function towards a healthier skin for babies.

The present invention thus provides for a composition for therapeutic prevention of growth of harmful bacteria on skin. Model harmful bacteria which have been tested and observed to be inhibited include *E. coli, S. aureus,* or mixtures thereof.

The invention thus provides for a composition that contains prebiotic glycerol. In other words the invention provides for therapeutic use of glycerol that has been proven to be a prebiotic. The advantages of the invention therefore are that it provides for therapeutic use of prebiotic glycerine that nourishes skin microbiome especially of babies, helps fight harmful aggressors and protects the skin from infections and helps to deliver prebiotic moisture or prebiotic moisturization. In another sense, the invention is capable of caring for baby's skin. One way that the invention does this is by providing a protective barrier on skin using the body's natural microbiome and through use of prebiotics to feed the natural microbiome. This way, the present invention develops a healthy and resilient skin and improve and build a better barrier. Another advantage of the present invention is that it does not disrupt the skin pH, which plays an important role in microbiome health, and replenishes skin with essential nutrients to help skin retain its natural moisture.

Another aspect of the present invention relates to a composition comprising the sugar or sugar alcohol for therapeutic use in protecting skin from harmful bacteria while nourishing and growing the skin commensal bacteria wherein the sugar is selected from monosaccharides glucose, fructose, galactose, or disaccharides sucrose, lactose; or sugar alcohols glycerol, lactitol. The composition is a composition for use in therapeutically protecting skin from harmful bacteria via nourishing the skin commensal bacteria.

The invention thus provides for nourishing the good microbes thus maintaining the health of the microbiome. The invention thus helps in maintaining the host microenvironment to help the right microbes flourish. It does this by maintaining the pH mantle of the host. Alternatively, the present invention ensures that the microbiome balancing is obtained by selectively reducing the number of harm causing microbes residing on the external surface like skin whilst helping the beneficial microbes thrive. Thus, the invention provides for a composition comprising sugar or sugar alcohol and the metabolites produced therefrom by the skin commensal bacteria to deliver a beneficial effect on skin health.

The invention will now be demonstrated with the help of the following non-limiting examples.

### Examples

### Identification of fermentation products of glycerol using GC-MS:

GC-MS was used as a tool to identify and quantify the trace level metabolites that are produced as a result of glycerol fermentation. SPME (solid phase extraction) was used for the extraction of metabolites from the aqueous supernatants. The GC-MS chromatogram of S. *epidermidis* 12228 grown in the presence and absence of glycerol and the respective media control was analyzed. The chromatogram profile was different for *S*. *epidermidis* 12228 grown in the presence of glycerol compared to the one without glycerol and the respective media control. The presence of higher number of peaks, indicated the formation of new metabolites in the presence of glycerol.

The GC-MS chromatogram of three other *Staphylococcus* Spp. (grown in the presence of glycerol) were compared with *S. epidermidis* 12228. The GC-MS fingerprint profiles indicated no metabolites produces by the two species *S*. *hominis* and S. *capitis.* Whereas there are higher number of metabolites detected from both *S. epidermidis* 12228 and S.

### epidermidis 1457

The metabolites produced by the two species *S. epidermidis* 12228 and *S. epidermidis* 1457 were identified using NIST and Wiley library available with GC-MS instrument.

The metabolites identified includes fatty acids. Since organic acids are not volatile, a second method has been developed to quantify them which involves a derivatization approach. The key organic acids identified in these samples were lactic acid and succinic acid. It was observed that the level of lactic acid, an alpha hydroxy acid (AHA) is higher than short chain fatty acids (SCFAs). The above classes of metabolites were quantified using GC-MS to clearly distinguish the degree of glycerol fermentation by different *Staphylococcus* epidermidis strains.

Using a targeted GC-MS approach, organic acids were quantified. Among all the metabolites, lactic acid was found at a higher level in *S. epidermidis* 12228 compared to other species. This finding suggest that lactic acid might be the key metabolite responsible for the inhibitory activity of ferments from *S epidermidis.* Thus, the GCMS fingerprint clearly distinguished all the *Staphylococcus* species in terms of fermentation ability of glycerol and the key metabolites produced by fermentation.

*In-vivo* production of lactic acid by application of glycerol: Dose dependent increase in Lactic acid levels upon application of pure glycerol on forearm of human volunteers:
From *in-vitro* studies, lactic acid was identified as the key metabolite of glycerol fermentation by *S epidermidis,* the common skin commensal. To test whether this also happens on skin, in *vivo* POP studies were done on human volunteers to check for the increase in lactic acid production. Pure glycerol (at different concentrations 2.5%, 5% and 10%) was applied on forearm of human volunteers thrice a day for two days and lactic acid levels were quantified using GC-MS. Dose dependent increase in lactic acid levels was observed upon application of increased concentrations of glycerol compared to control (no application) indicating that glycerol is being utilized by commensals present on skin (forearm) to produce lactic acid.

### Inhibition of E. coli and S. aureus by Lactic acid:

Analysis of the metabolite profile of glycerol ferments by *S*. *epidermidis* indicated lactic acid as the major metabolite produced. In this experiment the effect of pure lactic acid on growth of both commensal and harmful bacteria was tested. The growth kinetics of S. *epidermidis, S. aureus* and *E. coli* were monitored in presence of lactic acid at 0.25% and 0.5% at pH 4.6.

It was observed that the growth kinetics of both *E. coli* and S. *aureus* was severely affected in presence of lactic acid. The antimicrobial activity of lactic acid was clearly seen at pH 4.6 against both the pathogens at the concentrations tested. However, the antimicrobial effect of lactic acid was minimal against S. *epidermidis.* The above data indicates that glycerol ferments from *S epidermidis* inhibited growth of *E. coli* and S. *aureus.*

### Inhibition of S. aureus and E. coli by S. epidermidis in presence of 2% -glycerol:

To examine if glycerol fermentation by S. *epidermidis* resulted in inhibition of S. *aureus,* a pathogen present on skin the growth inhibition assay was performed. *S. epidermidis* 12228 was grown in TSB with and without glycerol (20g/l) and ferments were collected after 72hrs. These ferments were diluted in TSB at 50% dilution and growth kinetics of *S. aureus* was monitored. It was observed that growth of S. *aureus* was compromised in presence of glycerol ferment as compared to control suggesting that the metabolites present in the ferment inhibited its growth. The TSB filtrate also showed slight inhibitory effect on growth of *S*. *aureus;* this might be due to various metabolites produced by S. *epidermidis.*

The ability of these ferments to inhibit growth of gram-negative bacteria was also tested. The growth kinetics of *E. coli* was monitored in presence of these ferments using the same protocol that was used against *S*. *aureus.* The growth rate of *E. coli* in presence of control ferment remain unchanged but it was clearly compromised in presence of glycerol ferment. This suggests that the by-products of glycerol fermentation from *S. epidermidis* can inhibit both gram-positive and gram-negative pathogens.

Direct evidence of inhibition of S. *aureus by S. epidermidis* in the absence of glycerol or in the presence of 1% glycerol:

The objective of this experiment was to show by a direct experiment that the glycerol fermentation by *S epidermidis* can inhibit the growth of S aureus.

Two petridishes were taken. In one petridish *S. epidermidis* was grown along with glycerol and in the other petridish it was grown in the absence of glycerol. After 24 hours of growth, *S aureus* was added on top of it in "Spots". The spots were visually observed after 24 hours. It was evident from the visual inspection that the petridish where glycerol was added, the growth of S aureus was inhibited (there were less colonies) compared to the plates where there was no glycerol. This shows that in the presence of the prebiotic (glycerol) and the commensal bacteria S epidermidis, the harmful bacteria S aureus's growth is inhibited.

Sample compositions as per the invention which provide the benefits claimed herein are given below:
Example - 1: Baby lotion

| Ingredient | Wt% |
|---|---|
| Glycerine | 12.0 |
| Petrolatum | 6.5 |
| Stearic Acid | 2.5 |
| Glycol Stearate | 1.5 |
| Dimethicone | 1.0 |
| Isopropyl Isostearate | 1.0 |
| Tapioca Starch | 1.0 |
| Triethanolamine | 0.9 |
| Water | To 100 |

Example - 2: Moisturising Bodywash composition

| Ingredient | Wt% |
|---|---|
| Stearic Acid | 0.3 |
| Caprylyl glycol | 0.5 |
| Ultrez 20 | 0.8 |
| Sodium Methyl Lauroyl Taurate | 0.5 |
| Sodium Lauroyl Glutamate | 2.5 |
| CAPB (Betaine) | 3.0 |
| Glycerol | 3.5 |
| Sodium Benzoate | 0.5 |
| Water | To 100 |

Example - 3: Mild bodywash composition

| | Wt% |
|---|---|
| SLES(sodium lauryl ether surfactant) | 6.75 |
| CAPB (cocoamidipropyl betaine) | 2.75 |
| Glycerin | 70.00 |
| Water | To 100 |

Example - 4: Foaming mild cleansing composition

| | Wt% |
|---|---|
| Sodium cocoamidopropyl betaine | 2.25 |
| Sodium lauroyl glutamate | 6.75 |
| Glycerin | 60.00 |
| Guar Hydroxypropyltrimonium Chloride | 0.10 |
| Xanthan gum | 1.0 |
| Keltro CG-SFT | |
| Deionized water | To 100 |

Example - 5: Self foaming cleansing composition

| | Wt% |
|---|---|
| Glutamate (Sodium lauroyl glutamate) | 11.25 |
| CAPB (cocoamidipropyl betaine) | 3.75 |
| Glycerine | 20.0 |
| Water | To balance |

## Claims

1. A sugar or sugar alcohol, wherein the sugar is selected from monosaccharides glucose, fructose, galactose, or disaccharides sucrose, lactose; or sugar alcohols glycerol, lactitol, for use in therapeutically inhibiting growth of harmful bacteria while nourishing and growing the skin commensal bacteria.
wherein the harmful bacteria are selected from *E. coli, S. aureus* or mixtures thereof,
wherein the sugar or sugar alcohol is a prebiotic for the skin commensal bacterium to produce lactic acid,
wherein the skin commensal bacterium is *S. epidermidis.*

2. A sugar or sugar alcohol for use according to claim 1, wherein the sugar alcohol is glycerol.

3. A sugar or sugar alcohol for use as claimed in claim 1 or 2, in a composition comprising from 0.1 to 80% of the sugar or sugar alcohol by weight of the composition.

4. A sugar or sugar alcohol for use as claimed in any one of claims 1 to 3 in a composition substantially free of a conventional antimicrobial compound.

## Patentansprüche

1. Zucker oder Zuckeralkohol, wobei der Zucker unter den Monosacchariden Glucose, Fructose, Galactose oder den Disacchariden Saccharose, Lactose oder den Zuckeralkoholen Glycerin, Lactitol ausgewählt ist, zur Verwendung bei der therapeutischen Hemmung des Wachstums schädlicher Bakterien, während die hautkommensalen Bakterien ernährt werden und wachsen,
wobei die schädlichen Bakterien unter *E. coli, S. aureus* oder Mischungen davon ausgewählt sind, wobei der Zucker oder Zuckeralkohol ein Präbiotikum für das hautkommensale Bakterium ist, um Milchsäure zu produzieren,
wobei das hautkommensale Bakterium *S. epidermidis* ist.

2. Zucker oder Zuckeralkohol zur Verwendung gemäß Anspruch 1, wobei der Zuckeralkohol Glycerin ist.

3. Zucker oder Zuckeralkohol zur Verwendung gemäß Anspruch 1 oder 2 in einer Zusammensetzung, die 0,1 bis 80 % des Zuckers oder Zuckeralkohols, bezogen auf das Gewicht der Zusammensetzung, umfasst.

4. Zucker oder Zuckeralkohol zur Verwendung gemäß einem der Ansprüche 1 bis 3 in einer Zusammensetzung, die im Wesentlichen frei von einer herkömmlichen antimikrobiellen Verbindung ist.

## Revendications

1. Sucre ou alcool de sucre, où le sucre est choisi parmi les monosaccharides glucose, fructose, galactose ou les disaccharides saccharose, lactose; ou les alcools de sucre glycérol, lactitol, destiné à être utilisé pour inhiber thérapeutiquement la croissance de bactéries nocives tout en nourrissant et faisant croître les bactéries commensales cutanées,
où les bactéries nocives sont choisies parmi *E. coli, S. aureus* ou leurs mélanges,
où le sucre ou l'alcool de sucre est un prébiotique pour que la bactérie commensale cutanée produise de l'acide lactique,
où la bactérie commensale cutanée est *S. epidermidis.*

2. Sucre ou alcool de sucre destiné à être utilisé selon la revendication 1, où l'alcool de sucre est le glycérol.

3. Sucre ou alcool de sucre destiné à être utilisé selon la revendication 1 ou 2, dans une composition comprenant de 0,1 à 80 % de sucre ou d'alcool de sucre en poids de la composition.

4. Sucre ou alcool de sucre destiné à être utilisé selon l'une quelconque des revendications 1 à 3 dans une composition sensiblement exempte de composé antimicrobien conventionnel.
